# EUROPÄISCHE PATENTANMELDUNG

(11) **EP 3 031 443 A1**
(43) Veröffentlichungstag der Anmeldung: **15.06.2016**
(21) Anmeldenummer: 14197689.4
(22) Anmeldetag: 12.12.2014
(51) Int. Cl.: A61K 8/00

(54) **Zusammensetzung enthaltend Kohlehydratpartialester**

(71) Anmelder: BASF SE, 67056 Ludwigshafen am Rhein (DE)
(72) Erfinder: Busch, Stefan, 40597 Düsseldorf (DE); Mahnke, Eike Ulf, 42553 Velbert (DE); Machado Sincero, Melina, 40545 Düsseldorf (DE); Kloeker, Markus, 40233 Düsseldorf (DE)
(74) Vertreter: Fitzner, Uwe

(57) **Zusammenfassung**

Die vorliegende Erfindung betrifft eine Zusammensetzung enthaltend Kohlehydratpartialester, deren Herstellung sowie Erzeugnisse enthaltend die erfindungsgemäßen Kohlehydratpartialester.

## Beschreibung

Die vorliegende Erfindung betrifft eine Zusammensetzung enthaltend Kohlehydratpartialester, deren Herstellung sowie Erzeugnisse enthaltend die erfindungsgemäßen Kohlehydratpartialester.

Verfahren zur Herstellung von Kohlehydratpartialestern sind aus der EP 1 811 951 A1, EP 0 885 898 B1, GB 1 399 053 sowie GB 1 499 989 bekannt. Die Herstellung beruht im Wesentlichen auf einer katalysierten Umesterung von Kohlehydraten mit Fettsäurealkylestern oder Glyceriden. Die Aufreinigung oder Aufarbeitung des Rohprodukts nach der Umesterung wird in der WO 90 11285 A oder JP 383 6162 B2 beschrieben.

Die Kohlehydratpartialester werden üblicherweise in Gegenwart eines alkalischen Katalysators durch Umesterung von Glykosen mit Fettsäuremethylestern bei Temperaturen von 120 - 160°C hergestellt. Nach Zugabe von Lösungsmitteln und/oder Emollients erfolgt eine Abtrennung des nicht umgesetzten Zuckers nach Abkühlung auf 60 - 130°C durch Dekantieren und Filtration.

Für eine effiziente Aufarbeitung werden dem Rohprodukt üblicherweise Hilfsstoffe zugesetzt, die zu einer Verringerung der Viskosität führen und dadurch auch bei geringeren Temperaturen eine schnelle Abtrennung des Restzuckers durch Dekantieren und Filtrieren ermöglichen. Hier werden bevorzugt inerte Kohlenwasserstoffe wie hydriertes Polyisobuten eingesetzt. Sofern die zur Verringerung der Viskosität eingesetzten Hilfsstoffe (Emollients) unter den Aufbereitungsbedingungen reaktiv sind, ist eine vollständige Deaktivierung des Umesterungskatalysators erforderlich. Unter den Aufarbeitungsbedingungen reaktiver Emollients sind beispielsweise Fettalkohole oder Esteröle. Sofern der Katalysator nicht vollständig deaktiviert ist, finden weitere Umesterungsreaktionen statt, so dass eine Veränderung der Verteilung der Kohlehydratpartialester festzustellen ist.

Ferner liegen in dem Rohprodukt auch nicht umgesetzte Fettsäureester wie z.B. die oben bezeichneten Fettsäuremethylester oder andere Esteröle mit kurzkettigen Alkoholkomponenten vor, die aufgrund ihrer Reaktivität als Edukte bei der Synthese von Kohlehydratpartialestern eingesetzt werden.

Die Verwendung von Estern mit längerkettigen Alkoholkomponenten als Emollients senkt einerseits die Reaktivität gegenüber einer Umesterung während der Aufarbeitung, andererseits wird aber die Viskosität erhöht. Durch die Erhöhung der Viskosität muss die Aufarbeitung, insbesondere die Filtration, bei deutlich höheren Temperaturen erfolgen. Um eine höhere Temperatur zu vermeiden und die Viskosität zu senken, muss der Anteil an Emollients weiter erhöht werden. Sowohl eine Erhöhung der Viskosität als auch eine Erhöhung des Anteils an Emollients verringern die Effizienz der Aufarbeitungsschritte. Allgemein beeinflussen Emollients aus Kohlenwasserstoffen die Eigenschaften von Pastillen negativ.

Des Weiteren ist aus prozesstechnischer Sicht eine vollständige Deaktivierung des Katalysators schwierig zu erreichen und stellt einen zusätzlichen Prozessschritt dar. Da üblicherweise alkalische Umesterungskatalysatoren eingesetzt werden, erfolgt die Deaktivierung durch Säurezugabe und gelingt häufig nur unvollständig.

Die Zugabe von Hilfsstoffen wie z.B. die oben erwähnten zusätzlichen Emollients oder Säure beeinflussen die Farbe und den Säuregehalt des Rohprodukts negativ.

Aufgabe der vorliegenden Erfindung war es, die Nachteile des Standes der Technik, insbesondere bezüglich der Aufarbeitung des Rohprodukts, zu überwinden.

Hierzu sollte ein Verfahren zur Herstellung von Kohlehydratpartialester sowie zur Aufarbeitung des Rohprodukts zur Verfügung gestellt werden, das durch eine geringe Anzahl von Arbeitsschritten hoch effizient ist. Dies sollte durch ein lösungsmittelfreies und abfallarmes Verfahren erfolgen.

Von besonderer Bedeutung war dabei, dass sich die Verteilung der Kohlehydratpartialester des Rohprodukts nach dem Beenden der Synthese des Kohlehydratpartialestergemischs nicht von der Verteilung der Kohlehydratpartialester im aufgereinigten bzw. aufgearbeiteten Endprodukt unterscheidet. In dem Verfahren soll außerdem durch die eingesetzten Emollients eine Viskosität erreicht werden, die einerseits eine effiziente Abtrennung des Restzuckers (nicht umgesetzte Kohlenhydrate) durch Filtration und/oder Dekantierung, bevorzugt in nur einem Auftrennungsschritt, ermöglicht. Andererseits soll ein (partielles) Lösen des Restzuckers in größeren Mengen aufgrund der im Vergleich zu Kohlenwasserstoffen höheren Polarität des Verdünners vermieden werden.

Weitere Aufgabe der vorliegenden Erfindung war es, eine Kohlehydratpartialester enthaltende Zusammensetzung zur Verfügung zu stellen. Die Zusammensetzung soll eine genau definierte Partialesterverteilung besitzen, die sich durch Aufarbeitung oder Lagerung nicht ändert. Des Weiteren soll die Zusammensetzung bezüglich Farbe, Stabilität und Viskosität alle Anforderungen für eine unmittelbare Weiterverarbeitung erfüllen, insbesondere für eine Verwendung in kosmetischen oder pharmazeutischen Formulierungen. Bevorzugt soll die Zusammensetzung im Wesentlichen oder ausschließlich aus nachhaltigen Rohstoffen bestehen. Kohlenwasserstoffe petrochemischen Ursprungs sollen vermieden werden bzw. nicht darin enthalten sein. Außerdem sollen Formkörper enthaltend die erfindungsgemäße Zusammensetzung oder im Wesentlichen daraus bestehend eine bessere Lagerstabilität hinsichtlich Konsistenz, Farbe und Geruch aufweisen. Die Formkörper müssen ausreichend hart sein und sollen nicht zusammen kleben, um ein Zusammenbacken auch bei längerer Lagerung zu vermeiden. Auch unter ungünstigen Lagerbedingungen wie z.B. erhöhter Temperatur darf das Emollient nicht aus dem Formkörper austreten. Spröde Formkörper haben eine reduzierte Stabilität und führen zu Staubbildung. Eine Zugabe von zusätzlichen Hilfsstoffen, insbesondere zur Stabilisierung der Formkörper, soll vermieden werden.

Diese Aufgaben werden durch Zusammensetzung, Formkörper und das Verfahren gemäß der Ansprüche gelöst.

Gegenstand der vorliegenden Erfindung ist eine Zusammensetzung enthaltend:
a1) Kohlehydratpartialester als Produkt einer Veresterung mindestens eines Kohlehydrats mit Acylkomponenten der Formel R1-COO,
b1) Alkylester der Formel R1-COO-R2 und
c1) Esteröle der Formel R1-COO-R3.
sowie ggf. eine oder mehrere Verbindungen ausgewählt aus der Gruppe enthaltend oder bestehend aus: Kohlehydrate, Fettsäuren, Fettseifen, Wasser und Katalysator;
wobei die Esteröle c1) eine Alkoholkomponente R3 ausgewählt aus der Gruppe der
i) verzweigten und unverzweigten Alkoholen mit C8 - C22, bevorzugt C10 - C20, besonders bevorzugt C12 oder C14 bis C20, insbesondere C16 und/oder C18oder
ii) Polyalkoholen oder
iii) Mischungen davon
   besitzen, welche vollständig verestert sind mit einer Acylkomponente R1 ausgewählt aus der Gruppe von Mono- und Disäuren der Kettenlänge C6 - C22, bevorzugt C10 - C20, besonders bevorzugt C12 oder C14 bis C20, insbesondere C16 und/oder C18 oder beliebige Kombinationen.

Weiterer Gegenstand der vorliegenden Erfindung ist eine Zusammensetzung enthaltend:
a2) Kohlehydratpartialester mit einem durchschnittlichen Veresterungsgrad von 1-4,
b2) organische Alkylester mit Acylkomponente mit 6 - 22 Kohlenstoffatomen, bevorzugt C10 - C20, besonders bevorzugt C12 oder C14 bis C20, insbesondere C16 und/oder C18,
c2) Esteröle mit einer linearen Acylkomponente mit 6-22 Kohlenstoffatomen, bevorzugt C10 - C20, besonders bevorzugt C12 oder C14 bis C20, insbesondere C16 und/oder C18 und einer linearen Alkoholkomponente mit 8 - 22 Kohlenstoffatomen, bevorzugt C10 - C20, besonders bevorzugt C12 oder C14 bis C20, insbesondere C16 und/oder C18,
   sowie ggf. eine oder mehrere Verbindungen ausgewählt aus der Gruppe enthaltend oder bestehend aus: Kohlehydrate, Fettsäuren, Fettseifen, Wasser und Katalysator.

In einer Ausführung enthält die erfindungsgemäße Zusammensetzung:
a) Kohlehydratpartialester mit einem durchschnittlichen Veresterungsgrad von 1 - 4 in einem Anteil von 50-90%, bevorzugt 55-80%, besonders bevorzugt 55-70%, insbesondere 55-65%;
b) organische Alkylester mit Acylkomponente mit 6 - 30 Kohlenstoffatomen in einem Anteil von 0-10%, bevorzugt 2,5-7,5%, besonders bevorzugt 3-5%, insbesondere 4% oder 5%;
c) Esteröle in einem Anteil von 5-30%, bevorzugt 10-20%, besonders bevorzugt 12-17%, insbesondere 15%;
   sowie ggf. Kohlehydrate (Restzucker) in einem Anteil von 0-10%, bevorzugt 3-8%, insbesondere 3-5%; und/oder
   ggf. Fettseifen in einem Anteil 0-20%, bevorzugt 1-15%, besonders bevorzugt 5-12%, insbesondere 10% oder 12% und/oder ggf.

Fettsäuren: 0-10%, bevorzugt 2,5-7,5, besonders bevorzugt 3-5%, insbesondere 4% oder 7%.

Die Verbindungen bzw. Komponenten a), b) und c) stehen jeweils für a1) und/oder a2), b1) und/oder b2) bzw. c1) und/oder c2).

Die erfindungsgemässe Zusammensetzung kann Wasser in einem Anteil von 5%, bevorzugt 2%, besonders bevorzugt 1%, insbesondere 0.5 -1% oder weniger enthalten oder im Wesentlichen oder völlig wasserfrei sein. »Im Wesentlichen wasserfrei« bedeutet, dass der Wassergehalt der Zusammmensetzung < 5 Gew.-%, vorzugsweise < 2 Gew.-%, bevorzugt < 1 Gew.-% und insbesondere < 0,1 Gew.-% ist. »Völlig wasserfrei« bedeutet, dass der Wassergehalt unterhalb der Nachweisgrenzen der üblichen und bekannten Methoden zur quantitativen Bestimmung von Wasser liegt.

Die erfindungsgemässe Zusammensetzung kann Kohlenhydrate (Restzucker) in einem Anteil von 5%, bevorzugt 2%, besonders bevorzugt 1%, insbesondere 0.5% oder weniger enthalten oder im Wesentlichen oder völlig Kohlehydrat-frei sein. »Im Wesentlichen Kohlehydrat-frei« bedeutet, dass der Kohlehydratgehalt der Zusammensetzung < 5 Gew.-%, vorzugsweise < 2 Gew.-%, bevorzugt < 1 Gew.-% und insbesondere < 0,1 Gew.-% ist. »Völlig Kohlehydrat-frei« bedeutet, dass der Kohlehydratgehalt unterhalb der Nachweisgrenzen der üblichen und bekannten Methoden zur quantitativen Bestimmung von Wasser liegt.

Die Summe der Verbindungen bzw. der Komponenten der erfindungsgemässen Zusammensetzung beträgt jeweils 100%.

In einer Ausführung ist das Produkt a1) oder a2) ein Kohlehydratpartialester einer Glykose mit Acylkomponente von b1) oder b2) ist.

Unter den Glykosen werden die auch als Kohlenhydrate bezeichnete Polyhydroxyaldehyde (Aldosen) und Polyhydroxyketone (Ketosen) sowie höhermolekulare Verbindungen zusammengefasst, die sich durch Hydrolyse in solche Stoffe überführen lassen. Im Sinne der Erfindung können als Glykosen sowohl die monomeren Polyhydroxyaldehyde oder Polyhydroxyketone (Monosaccharide) oder ihre Dimeren bis Decameren (Disaccharide, Trisaccharide, Oligosaccharide) eingesetzt werden. Als Monosaccharide (auch "einfache Zucker" genannt) kommen beispielsweise Biosen, Triosen, Tetraosen, Pentosen, Hexosen, Heptosen etc. in Frage. Typische Beispiele für Aldopentosen sind D-Ribose, D-Xylose und L-Arabinose. Zu den wichtigsten Aldohexosen gehören D-Glucose, D-Mannose und D-Galactose; bei den Ketohexosen sind D-Fructose und Sorbose zu nennen. Die 6-Desoxyzucker L-Fucose und L-Rhamnose sind ebenfalls weit verbreitete Hexosen und kommen als Ausgangsstoffe ebenfalls in Frage. Die einfachsten Oligosaccharide, die als Ausgangsstoffe geeignet sind, stellen die Disaccharide dar. Vorzugsweise werden Saccharose (Rohrzucker, Rübenzucker), Lactose (Milchzucker) und/oder Maltose (Malzzucker) eingesetzt.

Der Einsatz von Mono- und/oder Disacchariden ist im Sinne des Verfahrens bevorzugt; insbesondere werden Saccharose (Sucrose) oder Glucose bevorzugt eingesetzt.

In einer Ausführung der vorliegenden Erfindung ist die Alkoholkomponente des Esters b1) oder b2) eine C1 bis C3 Alkoholeinheit, bevorzugt Methyl und/oder Ethyl.

In einer Ausführung wird mindestens ein Alkylester b2) und/oder b1) mit einer Acylkomponente ausgewählt aus der Gruppe aliphatischen, linearen oder verzweigten Acylresten mit 6 bis 22 Kohlenstoffatomen und 0 und/oder 1, 2 oder 3 Doppelbindungen enthalten. Ferner ist die Acylkomponente ausgewählt aus der Gruppe enthaltend die Acylreste von Capronsäure, Caprylsäure, 2- Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen mit Methanol, Ethanol, Propanol, Isopropylalkohol, n-Butanol, i-Butanol, tert.Butanol, n-Pentanol und Isopentanol oder deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen, wie beispielsweise Kokos-, Palm-, Palmkern- oder Talgfettsäure.

In einer Variante werden Alkylester b1) und/oder b2) mit linearen, gesättigten Acylresten mit 12 bis 30C-Atomen, vorzugsweise mit 16 bis 24 C-Atomen eingesetzt. Bevorzugt sind C16-C24-Fettsäuremethylester, insbesondere C16-C20-Fettsäuremethylester, wobei lineare und unverzweigte Ester unter diesen erfindungsgemäss als besonders bevorzugt gelten. Erfindungsgemäss vorteilhaft ist der Einsatz von C16-Fettsäuremethylestern oder C18-Fettsäuremethylestern oder von beliebigen Gemischen von C16/C18-Fettsäuremethylestern, beispielsweise mit einem C16/C18-Anteil von 50:50, 40:60, 30:70, 60:40 oder 70:30.

In einer Variante wird ein Ester der Isostearinsäure eingesetzt.

Das molare Einsatzverhältnis von Alkylester : Kohlehydrat beträgt vorzugsweise wenigstens 0,5, insbesondere 0,5 - 2,5 und besonders bevorzugt 0,6 - 2,0. Bei geringeren Molverhältnissen sind die Reaktionsprodukte zunehmend gefärbt und viskos, was vermutlich auf eine Karamelisierung des Zuckers zurückzuführen ist. Erfindungsgemäss besonders bevorzugt ist ein Molverhältnis von 1,3 - 1,6, insbesondere 1,4 - 1,55 um eine Karamelisierung des Zuckers zu reduzieren und farbliche hellere Rohprodukte zu erhalten.

In einer weiteren Ausführung ist mindestens eine Acylkomponente des Esters a1), b1), c1), a2), b2) oder c2) ein Fettsäurerest mit 16 oder 18 Kohlenstoffatomen.

In einer Alternative sind alle Acylkomponenten der Ester a1), b1), c1), a2), b2) oder c2) identisch.

In einer weiteren Alternative sind alle Acylkomponenten der Ester a1), b1), c1), a2), b2) oder c2) Fettsäurereste mit 16 oder 18 Kohlenstoffatomen, bevorzugt sind alle identisch.

Eine Ausführung der vorliegenden Erfindung betrifft eine Zusammensetzung dadurch gekennzeichnet, dass die Alkoholkomponente der Esteröle c1) oder c2) ein Fettalkohol mit 16 und/oder 18 Kohlenstoffatomen oder ein Guerbetalkohol mit 16 bis 20 Kohlenstoffatomen ist.

In einer Alternative wird als Esteröl c1) und/oder c2) Cetyl Palmitat (zum Beispiel Cutina CP) eingesetzt.

In einer weiteren Alternative werden als Esteröle c1) Cetyl Palmitat (zum Beispiel Cutina CP), Guerbet Hexyldecyl Stearat (zum Beispiel Eutanol G16S) und/oder gehärtetes Palmfett Triglycerid (zum Beispiel Waretta 863) eingesetzt. Gehärtetes Palmfett wird durch (partielle) Hydrierung der Doppelbindungen ungesättigter Fettsäuren im entsprechenden Öl gewonnen und zeichnet sich u.a. durch einen erhöhten Schmelzpunkt und verbesserte Oxidationsstabilität aus.

Eine Alternative der Zusammensetzung ist dadurch gekennzeichnet, dass die Alkoholkomponente der Esteröle c1) oder c2) ein Polyalkohol ausgewählt aus der Gruppe enthaltend / bestehend aus: Ethylenglykol, Propylenglykol, Butylenglykol, Neopentylglykolen (NPG, TMP, PE), Glycerin ist. Als Neopentylglykole können 2,2-Dimethyl-1,3-propandiol = NPG, Trimethylolpropan = TMP oder Pentaerythrit = PE eingesetzt werden.

Bevorzugt sind die Polyalkohole vollständig verestert. In einer Ausführung wird Glycerin vollständig verestert eingesetzt.

In einer Variante können erfindungsgemäß als Alkoholkomponente aliphatische, lineare oder verzweigte Kohlenwasserstoffrest mit 6 bis 22 Kohlenstoffatomen und 0 und/oder 1, 2 oder 3 Doppelbindungen eingesetzt werden.

Bevorzugt werden Fett- und/oder Guerbetalkohole ausgewählt aus der Gruppe enthaltend oder bestehend aus: C16- und/oder C18-Fettalkohol, C16-, C18- und/oder C20-Guerbetalkohol.

In einer Alternative wird die Alkoholkomponente ausgewählt aus der Gruppe enthaltend oder bestehend aus: Capronalkohol, Caprylalkohol, 2-Ethylhexylalkohol, Caprinalkohol, 2-Propylheptylalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol.

Die Zusammensetzung enthält in einer Variante keine freie Alkoholkomponente des Esteröls.

Die Zusammensetzung enthält in einer Variante keine freie Partialglyceride des Esteröls.

In einer weiteren Ausführung enthält die erfindungsgemäße Zusammensetzung unterschiedliche Kohlehydradpartialester.

In einer Alternative liegen
Monoester in einem Anteil von 0%-20%, bevorzugt 5-15%, besonders bevorzugt 7-12%, insbesondere 7% oder 8%;
Diester in einem Anteil von 10%-40%, bevorzugt 20-35%, besonders bevorzugt 25-30%, insbesondere 24% oder 27%;
Triester in einem Anteil von 20%-50%, bevorzugt 30-45%, besonders bevorzugt 35-40%, insbesondere33% oder 37% und

Tetraester und höhere Ester in einem Anteil von 15%-40%, bevorzugt 20-40%, besonders bevorzugt 25-35%, insbesondere 26 % oder 29% vor, wobei die Summe 100% ist.

In einer weiteren Alternative beträgt das Verhältnis von Monoester : Diester : Triester: Tetraester zwischen 0.5 und 1.5 : zwischen 3.0 und 4.0 : zwischen 4.0 und 5.0 : zwischen 3.0 und 4.0, bevorzugt 1 : 3,5 : 4,7 : 3,7, besonders bevorzugt 1:3,4:(4,6 oder 4,7):(3,6 oder 3,7).

In einer Ausführung wird die Kohlehydratpartialesterverteilung mittels GPC bestimmt. Bevorzugt erfolgt die Bestimmung wie folgt:
Prinzip: Die Probe wird in Tetrahydrofuran gelöst, mittels Flüssigchromatografie getrennt und mit einem RI Detektor detektiert. Die flächenprozentige Quantifizierung basiert auf der Methode der Auswertung in Anlehnung zur Ph.Eur. Monographie Glycerinmonostearat Typ II, Methode 2.2.30, sowie DGF C-VI 5b (02)
Geräte: HPLC System mit RI-Detektor z.B. Acquity System der Firma Waters; RI Detektor; Empower Auswertesoftware; 2 X PLgel 5µm 100 A 30cm * 8mm Firma Dr. Maisch
HPLC Bedingungen:

| | |
|---|---|
| Trennsäule | PLgel 5µm 100 A 30cm * 8mm Firma Dr. Maisch |
| Eluent | Tetrahydrofuran |
| Fluss | 1,0 mL/min bei 25°C isokratisch |
| Injektor | 50µl |
| Detektor | RI bei 40°C |

### Durchführung der Bestimmung:

Auf der Analysenwaage werden in 50 ml-Messkolben ca. 0,5 g der Probe analytisch genau eingewogen und mit Ameisensäure angesäuert. Mit Tetrahydrofuran wird der Messkolben auf Maß aufgefüllt. Durch Schütteln ist zu homogenisieren.

Die Viskosität der erfindungsgemäßen, aufgearbeiteten Zusammensetzung beträgt 0.5 bis 3 Pa s, bevorzugt 1.0 bis 2.0, besonders bevorzugt 1.2 bis 1.5, insbesondere 1.3 Pa s bei 100°C und einer Scherrate von 100/s.

Die Viskosität wird im dynamischen Fließgleichgewicht bei 100°C und einer Scherrate von 100/s bestimmt, in einer Variante mittels eines Rheometers V-VOR 120 (Kegel-Platte Viskosimeter mit 4° und 40 mm) der Firma Bohlin.

Weiterer Gegenstand der vorliegenden Erfindung ist ein Formkörper enthaltend oder bestehend aus der erfindungsgemäßen Zusammensetzung.

Die erfindungsgemäßen Formkörper können neben der erfindungsgemäßen Zusammensetzung weitere Kohlenhydratpartialester enthalten. In einer Alternative enthalten sie zusätzlich ein für kosmetische Anwendungen geeignetes Emollient für Kohlenhydratpartialester und/oder 1 - 10 Gew.-% eines C16-C40-Fettalkohols oder eines beliebigen Gemisches dieser Fettalkohole, wobei der Gehalt an weiteren Hilfs- und Zusatzstoffen höchstens 20 Gew.-% beträgt.

Kohlenhydratpartialester werden zur Weiterverarbeitung in einer Ausführung in einem C16-C40-Fettalkohol, vorzugsweise ein C18-C30 und besonders bevorzugt eine C20-C24-Fettalkohol oder ein beliebiges Gemisch dieser Fettalkohole gelöst.

In einer Alternative enthält die erfindungsgemäße Zusammensetzung und/oder der erfindungsgemäße Formkörper kein C16-C40-Fettalkohol, vorzugsweise kein C18-C30 und besonders bevorzugt kein C20-C24-Fettalkohol oder kein beliebiges Gemisch dieser Fettalkohole.

Eine bevorzugte Ausführungsform des Formkörpers enthält 10 - 95 Gew.-% eines Kohlenhydratpartialesters bzw. eines Gemisches aus Kohlehydratpartialestern a), 5 - 20 Gew.- % eines für kosmetische Anwendungen geeigneten Emollients, bevorzugt Esteröle c) und Hilfs-und Zusatzstoffe, wobei der Gehalt an weiteren Hilfs- und Zusatzstoffen höchstens 10 Gew.-% beträgt. Vorzugsweise beträgt der Gehalt an weiteren Hilfs- und Zusatzstoffen weniger als 10 Gew.-%, insbesondere weniger als 5 Gew.-% an der Gesamtzusammensetzung. Ganz besonders bevorzugt beschränkt sich der Anteil an weiteren Hilfs-und Zusatzstoffen lediglich auf Nebenprodukte, die reaktionsbedingt im Produktgemisch enthalten sind.

Die Formkörper sind lagerbeständig und zeigen kein "Schwitzen", auch bei Lagerung länger als eine Woche, bevorzugt 1 Monat, besonders bevorzugt 6 Monate, insbesondere 1 Jahr bei 40°C, d.h. es tritt keine Separierung flüssiger Produktbestandteile auf, die zur Bildung eines (durchgehenden) Flüssigkeitsfilms auf der Pastillenoberfläche oder an Behälterwänden führt. 'Schwitzen' ist unerwünscht, weil es die Verarbeitung des Produkt erschwert und dessen Aussehen beeinträchtigt.

Zur Untersuchung der Lagerstabilität wurden 15 ml Pastillen in einen Stahlzylinder gegeben und mit einem 900 g schweren Prüfstempel belastet und in dieser Anordnung einen Tag bei 40 °C gelagert. Bei guter Lagerstabilität zerfällt der erhaltene Probenkörper bei Raumtemperatur ohne große äußerliche Krafteinwirkung.

In einer Alternative handelt es sich bei dem Formkörper um eine Pastille und/oder um Schuppen.

Pastillen bestehen aus erstarrten Schmelzen oder festen Lösungen in einzeldosierter Form. Sie besitzen einen anderen Aufbau als Tabletten, die unter Pressdruck aus Pulvern oder Granulaten auf Tablettenpressen gefertigt werden, oder als Dragees, die eine mit einem Überzug versehene Formulierung darstellt. Pastillen werden durch Ausgießen einer Flüssigkeit in vorgefertigte Puderformen hergestellt. Danach werden die noch flüssigen Pastillen-Vorläufer schonend bis zum Erstarren gekühlt.

In einer bevorzugten Form wird die Schmelze des Produkts in einer Kühlbandanlage pastilliert, indem sie als Tropfen auf ein Band aufgetragen und während des Transports bis zur vollständigen Erstarrung gekühlt wird.

Eine weitere bevorzugte Form von Formkörpern stellen Schuppen dar. Die Verschuppung erfolgt beispielsweise mit einer Schuppenwalze: Die Schmelze wird in dünner Schicht auf eine gekühlte rotierende Walze aufgetragen, wo sie im Laufe der Rotation vollständig erstarrt. Das feste Produkt wird mittels feststehender Schaber kontinuierlich von der Walze gelöst und zerbricht dabei in kleine Platten, sogenannte Schuppen.

Ein weiterer Gegenstand der vorliegenden Erfindung ist ein Verfahren zur Herstellung der erfindungsgemäßen Zusammensetzung oder des erfindungsgemäßen Formkörpers gekennzeichnet durch folgende Schritte:
i) Veresterung eines Kohlehydrats mit einem Alkylester b1) oder b2) in Gegenwart eines Katalysators,
ii) Zugabe von Esterölen c1) oder c2),
iii) Abtrennung des Rohprodukts vom Restzucker, ggf.
iv) Bleichen der abgetrennten flüssigen Phase und ggf.
v) Herstellen eines Formkörpers.

Das erfindungsgemäße Verfahren kann in einer Alternative weitere Schritte umfassen.

In einer weiteren Alternative ist das Verfahren durch die oben beschriebenen Schritte charakterisiert und umfasst keine weiteren Schritte.

In einer Ausführung handelt es sich um ein solvensfreies Verfahren zur Herstellung von Kohlenhydratpartialestern mit einem durchschnittlichen Veresterungsgrad im Bereich von 1 bis 4 durch alkalikatalysierte Ver- bzw. Umesterung in Gegenwart eines Katalysator-Gemisches aus Alkalimetallcarbonat und Alkalimetallhypophosphit, wobei man
i-1) zur Bildung eines katalytisch aktiven Systems wenigstens ein Alkalimetallcarbonat und wenigstens ein Alkylester b1) und/oder b2) mischt und
i-2) zu der aus i-1) resultierenden Mischung unter stetigem Rühren Kohlehydrate bevorzugt mit 5 bis 12 Kohlenstoffatomen, gegebenenfalls Kohlenhydratpartialestern als Emulgatoren und Alkalimetallhypophosphit zugibt, so dass eine Dispersion resultiert, und dem resultierenden Gemisch unter stetigem Rühren bei Temperaturen bis zu 100°C bei einem Druck von bis zu 50 mbar den entsprechenden Alkylalkohol entzieht und
i-3) die Ver- bzw. Umesterungsreaktion bei einem Druck von bis zu 50 mbar und Temperaturen bis zu 125°C unter stetigem Rühren solange fortsetzt, bis der Gehalt an Alkylestem b1) und/oder b2) auf mindestens 8 Gew-% bezogen auf die Gesamtzusammensetzung abgefallen ist,
   wobei die Schritte i-1) bis i-3) optional unter einer Schutzgasatmosphäre durchgeführt werden.

Die Katalysator-Kombination aus Alkalimetallcarbonat und Alkalimetallhypophosphit liefert im erfindungsgemässen Verfahren farblich verbesserte Produkte in guten Ausbeuten und vergleichsweise kürzeren Reaktionszeiten, die einen geringeren Aschegehalt aufweisen. Zusätzlich wird die Bildung von Nebenprodukten, wie Seife reduziert. Als Schutzgas wird vorzugsweise Stickstoff eingesetzt. Der Einsatz von Schutzgasen liefert farblich noch hellere Produkte.

Die Reaktion findet in Abwesenheit von Lösungsmitteln statt, was sowohl aus ökonomischer Sicht als auch im Hinblick auf eine Verwendung im Kosmetiksektor von erheblichem Vorteil ist. Ein weiterer unerwarteter Vorteil des Verfahrens besteht darin, dass man trotz Reduktion des Alkalimetall-Katalysators eine effektive Umsetzung in vergleichbar kurzen Reaktionszeiten mit verbesserter Farbqualität erzielt.

In einer Alternative erfolgt das erfindungsgemäße Verfahren analog dem Verfahren beschrieben in der WO 2006/050832 A2, deren Offenbarung hiermit durch Bezugnahme eingebunden ist.

Als Katalysatoren wird erfindungsgemäss eine Kombination aus Alkalimetallcarbonate(n) und Alkalimetallhypophosphite(en) benutzt. Vorzugsweise wird Natrium- und/oder Kaliumcarbonat eingesetzt sowie Natrium- und/oder Kaliumhypophosphit. Erfindungsgemäss vorteilhaft ist es, 0,06 - 0,6 Mol Alkalimetallcarbonat und 0,01 - 0,1 Mol Alkalimetallhypophosphit pro Mol Glykose einzusetzten. Als erfindungsgemäss besonders vorteilhaft haben sich Einsatzmengen von 0,07 - 0,3 Mol Kaliumcarbonat und 0,01 - 0,05 Mol Natriumhypophosphit pro Mol Glykose, insbesondere 0,08 - 0,2 Mol Kaliumcarbonat und 0,01 - 0,03 Mol Natriumhypophosphit pro Mol Glykose, und ganz besonders bevorzugt 0,1 - 0,15 Mol Kaliumcarbonat und 0,012 - 0,02 Mol Natriumhypophosphit pro Mol Glykose erwiesen.

Im Sinne der Erfindung hat es sich als besonders vorteilhaft erwiesen als Emulgatoren Partialester von Kohlenhydraten einzusetzen, deren Kohlenhydrateinheit mit denen der Zielprodukte identisch ist. Erfindungsgemäss besonders vorteilhaft ist es, Kohlenhydratpartialester einzusetzen, in denen sowohl die Kohlenhydrateinheit als auch der Esterrest mit denen der Zielprodukte übereinstimmt, die sich also ggf. lediglich durch den Veresterungsgrad unterscheiden. Besonders bevorzugt ist der Einsatz von Saccharosepartialestern mit einem durchschnittlichen Veresterungsgrad im Bereich von 2 bis 6, insbesondere von 3 bis 4. Geeignete Zuckerester sind beispielsweise Sisterna (R) SP 01, Sisterna (R) SP 30 und Sisterna (R) SP 50. Erfindungsgemäss bevorzugt als Emulgatoren sind Zuckerester mit einem geringen Monoesteranteil, vorzugsweise weniger als 30 Gew.-% Monoesteranteil und insbesondere weniger als 1 Gew.-% Monoesteranteil. Erfindungsgemäss besonders bevorzugt ist der Einsatz von Partialester der Saccharose mit C16/C18-Fettsäuren, die einen entsprechend niedrigen Monoesterabteil aufweisen, da diese die Reaktionszeit herabsetzen und zu einer schnellen Umsetzung beitragen. Die Kohlenhydratpartialester können als Pulver, in flüssiger Form, aber auch in pelletierter Form eingesetzt werden. So hat es sich beispielsweise als vorteilhaft erwiesen, Pellets aus Saccharosepartialestem mit solchen Fettsäuremethylestern einzusetzen, die auch als Reaktanden verwendet werden.

Als zusätzliche Co-Emulgatoren kommen z.B. nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe sowie Alkylamine mit 8 bis 22 Kohlenstoffatomeen im Alkylrest;
(2) C2-C18-Fettsäuremono und -diester von Anlagerungsprodukten von 1 bis 30 Mol Ethylenoxid an Glycerin;
(3) Glycerinmono und -diester und Sorbitanmono und -diester von gesättigten und ungesättigten Fettsäuren mit 6 bis 22 Kohlenstoffatomen und deren Ethylenoxid-Anlagerungsprodukte;
(4) Alkylmono und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 1 bis 15 Mol Ethylenoxid an Ricinusöl und/oder gehärtetes Ricinusöl;
(8) Partialester auf Basis linearer, verzweigter, ungesättigter bzw. gesättigter C6/22-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Diund/oder Tri-PEG-alkylphosphate und deren Salze;
(10) Wollwachsalkohole;
(11) Polysiloxan-Polyalkyl-Polyether-Copolymere bzw. entsprechende Derivate; (12) Mischester aus Pentaerythrit, Fettsäuren, Citronensäure und Fettalkohol gemäss und/oder Mischester von Fettsäuren mit 6 bis 22 Kohlenstoffatomen, Methylglucose und Polyolen, vorzugsweise Glycerin sowie
(13) Polyalkylenglycole.

Das molare Einsatzverhältnis von Kohlenhydratpartialester als Emulgator : Kohlehydrat eingesetzt als Edukt beträgt vorzugsweise 0,03 - 0,25, vorzugsweise 0,04 - 0,2 und besonders bevorzugt 0,09 - 0,12.

Erfindungsgemäss bevorzugt ist ein Verfahren, bei dem das Molverhältnis von Alkalimetallcarbonat : Alkylester b1) und/oder b2) : Kohlehydrat eingesetzt als Edukt: Kohlenhydratpartialester als Emulgator : Alkalmetallhypophosphit im Bereich (0.06 - 0.6) : (0,6 - 2,0) : 1 : (0.04 - 0.2) : (0.01 - 0.1) variiert.

Die Reaktion wird dergestalt durchgeführt, dass unter starkem Rühren eine Emulsion/Dispersion hergestellt wird, in welcher das Katalysatorsystem, die Kohlehydrate und der Kohlenhydratester sowie gegebenenfalls weitere Emulgatoren enthalten sind. Das mechanische Rühren wird im Falle mittelbis hochviskoser Gemische im industriellen Massstab mit einem Rührsystem aus Impellerrührer in Kombination mit Stromstörer bei Umdrehungszahlen von 50 bis 400 Umdrehungen pro Minute, vorzugsweise 100-300 Umdrehungen pro Minute durchgeführt.

Vorzugsweise beträgt der Druck in Schritt i-2) und Schritt i-3) höchstens 25 mbar und ganz besonders bevorzugt höchstens 15 mbar, insbesondere höchstens 10 mbar. Durch diese Druckbedingungen wird sichergestellt, dass dem Reaktionsgleichgewicht möglichst effizient Wasser entzogen wird. Insbesondere soll die "Vortrocknung" in Schritt i-2) dazu dienen, das rohstoffbedingte Restwasser zu entziehen. Schritt i-2) wird vorzugsweise bei Temperaturen von 70 - 85°C, insbesondere 75 - 80°C und bei einem Druck von 1 bis 25 mbar, vorzugsweise 1 bis 15 mbar durchgeführt. Die eigentliche Veresterungsreaktion, Schritt i-3), wird vorzugsweise bei Temperaturen von 100°C - 125°C, insbesondere 110°C - 120°C, besonders bevorzugt 115°C - 120°C und bei einem Druck von 1 bis 25 mbar, vorzugsweise 1 bis 15 mbar und besonders bevorzugt 1 - 10 mbar durchgeführt. Die Veresterungsreaktion i-3) wird solange fortgesetzt, bis der Gehalt an Alkylestern b2) und/oder b1) auf mindestens 8 Gew-% bezogen auf die Gesamtzusammensetzung, vorzugsweise mindestens 5 Gew.-% bezogen auf die Gesamtzusammensetzung abgefallen ist. Die Reaktionszeiten liegen üblicherweise im Bereich von 5 bis 15 h und können durch effizientes Rühren erheblich reduziert werden.

Die so hergestellten Produkte weisen üblicherweise die oben beschriebene Kohlehydratpartialesterverteilung auf. Der Anteil nicht umgesetzter Glykosen im Rohprodukt liegt üblicherweise bei höchsten 15 Gew.-%, vorzugsweise bei höchsten 10 Gew.-%.

In einer weiteren Ausführungsform wird ein leichter Stickstoff-Schutzgasstrom durch die Reaktionsmischung geführt, ohne jedoch die oben angegebenen Druckbedingungen zu verlassen. Diese Verfahrensweise führt zu farblich helleren Produkten.

In einer erfindungsgemäss bevorzugten Verfahrensvariante wird das heisse Reaktionsprodukt in Esteröl c1) und/oder c2) als Emollient gelöst.

Nach Abtrennung der nicht umgesetzten Kohlehydrate wird mit Wasserstoffperoxid - vorzugsweise unter Stickstoffatmosphäre - gebleicht, gegebenenfalls zur Einstellung eines pH-Wertes zwischen 6 und 8 mit einer Säure behandelt wird und dann optional über ein Filtrationshilfsmittel filtriert wird.

In einer Alternative werden bei der Aufarbeitung keine weiteren Emollients eingesetzt.

In einer weiteren Alternative werden weder bei der Herstellung noch bei der Aufarbeitung weitere Emollients eingesetzt.

In einer Variante werden bei der Aufarbeitung weitere Emollients eingesetzt.

Unter weiteren Emollients im Sinne der Erfindung sind dabei Ölkörper zu verstehen, die bei 40°C, vorzugsweise bei 60°C und Normaldruck flüssig werden.

Die Emollients müssen die nach dem erfindungsgemässen Verfahren hergestellten Kohlenhydratpartialester lösen können, entweder bei Raumtemperatur oder ggf. in der Wärme. Als Emollient geeignet sind auch Ölkörper, die bei Raumtemperatur fest, pastös oder wachsartig, aber im geschmolzenen Zustand ein gutes Lösungsvermögen für die Kohlenhydratpartialester haben, bei das Erfindungsgemäss geeignet sind beispielsweise Kohlenwasserstoffe, Esteröle, Polyole, Dialkylether, Dialkylcarbonate, wie beispielsweise Cetiol(R) S, Sylko(R) 365 NF, Panalane(R) L 14 E, Cetiol(R) NPC, Cetiol(R) SN, Cetiol(R) PGL1 Edenor(R) V, Cetiol(R) OE, Cetiol(R) CC. Erfindungsgemäss bevorzugt geeignet sind Kohlenwasserstoffe und unter diesen ein bei 20°C und Normaldruck flüssiges Polyisobuten, insbesondere ein gehärtetes Polyisobuten, das unter der Bezeichnung Panalane(R) L14 E (Hersteller: Amoco; INCI-Bezeichnung: hydrogenated polyisobutene) im Handel ist. Letzteres zeichnet sich neben einer niedrigen Viskosität durch sehr gute Lösungseigenschaften für die erfindungsgemäss hergestellten Sucroseester und sensorische Vorteile bezüglich der kosmetischen Endformulierungen aus. Auch flüssige, pastöse oder wachsartige Esteröle aus C6-C22-Fettsäuren und C1-C3-Alkoholen wie z.B. Edenor(R) ME 16V sind als Lösungsmittel für erfindungsgemäss hergestellten Kohlenhydratpartialester gut geeignet.

In einer Ausführung erfolgt nach der Synthese der Kohlehydratpartialester weder in den weiteren Verfahrensschritten noch bei der Aufarbeitung eine Deaktivierung des Katalysators.

Die Abtrennung der nicht umgesetzten Kohlehydrate kann beispielsweise durch Dekantieren, Zentrifugieren oder durch Filtration erfolgen.

Erfindungsgemäss bevorzugt ist es, die Abtrennung durch Dekantieren vorzunehmen.

Wesentlich für das erfindungsgemässe Verfahren ist, dass das Produkt aus Schritt i) dieselbe Kohlehydratpartialesterverteilung aufweist wie eines der Produkte aus Schritt iii), iv) oder v). Das Zwischenprodukt, also Produkt aus Schritt i) ist viskos, trüb und dunkelbraun.

Nach der Abtrennung werden üblicherweise Zusammensetzungen erhalten, deren Restzuckergehalt kleiner 10 Gew.-%, insbesondere kleiner 5 Gew.-% bezogen auf die Gesamtzusammensetzung des Produktgemisches ist.

Anschließend erfolgt ein Bleichungsschritt mit Wasserstoffperoxid, der vorzugsweise unter Stickstoffatmosphäre durchgeführt wird. Das Reaktionsprodukt wird dann nochmals unter Vakuum getrocknet, um Restmengen an Wasser zu entfernen.

Der pH-Wert des Produktgemisches sollte zwischen 6 und 8 liegen und wird gegebenenfalls durch Zugabe von Säure eingestellt. Hierfür können die üblichen Mineralsäuren oder Fruchtsäuern eingesetzt werden. Erfindungsgemäss besonders geeignet ist die Zugabe von Zitronensäure oder Milchsäure. Milchsäure ist besonders geeignet, da die so neutralisierten Produkte zu stabileren kosmetischen Endformulierungen führen.

Mit dem erfindungsgemäßen Verfahren werden Kohlehydratpartialester in der gleichen Produktionszeit hergestellt wie im Verfahren der EP 1 811 951 B1. Die

Kohlehydratpartialesterverteilung des Rohproduktes ist mit jener des Endproduktes identisch.

Ein weiterer Gegenstand der vorliegenden Erfindung ist eine Zusammensetzung hergestellt in einem Verfahren wie oben beschrieben, bevorzugt gekennzeichnet durch folgende Schritte:
i) Veresterung eines Kohlehydrats mit einem Alkylester b) in Gegenwart eines Katalysators,
ii) Zugabe von Esterölen c),
iii) Abtrennung des Rohprodukts vom Restzucker, ggf.
iv) Bleichen der abgetrennten flüssigen Phase und ggf.
v) Herstellen eines Formkörpers.

Weitere Gegenstand der Erfindung sind kosmetische und/oder pharmazeutische Erzeugnisse oder Lebensmittel enthaltend eine erfindungsgemässe Zusammensetzung oder einen Formkörper oder ein Produkt, hergestellt in einem erfindungsgemässen Verfahren.

Kosmetische und/oder pharmazeutische Erzeugnisse im Sinne der Erfindung sind: Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben dienen.

Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe, Pigmente, Stoffe und/oder Pigmente mit farbgebenden Eigenschaften, Bakterizide bzw. bakteriostatische Wirkstoffe, schweißabsorbierende Substanzen und dergleichen enthalten.

Tenside: Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, alpha-Methylestersulfonae, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylgluamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217 verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, -Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen bzw. deren Salzen.

Ölkörper: Körperpflegemittel, wie Cremes, Lotionen und Milchen, enthalten üblicherweise eine Reihe weiterer Ölkörper und Emollients, die dazu beitragen, die sensorischen Eigenschaften weiter zu optimieren. Die Ölkörper können, ja nach Art der Formulierung in einer Gesamtmenge von 1 bis 90 Gew.-%, insbesondere in einer Gesamtmenge von 1 - 50 Gew.-%, vorzugsweise 5 - 25 Gew.-% und insbesondere 5 - 15 Gew.-% enthalten sein.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. DE 19756377 a2), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

Emulgatoren: Als Emulgatoren kommen beispielsweise nichtionogene Tenside aus mindestens einer der folgenden Gruppen in Frage:
➢ Alkyl- und/oder Alkenyloligoglykoside mit 8 bis 22 Kohlenstoffatomen im Alk(en)ylrest und deren ethoxylierte Analoga;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen z.B. Sorbit, Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin.;
➢ Block-Copolymere z.B. Polyethylenglycol-30 Dipolyhydroxystearate;
➢ Polymeremulgatoren, z.B. Pemulen-Typen (TR-1,TR-2) von Goodrich sowie
➢ Glycerincarbonat.

Polyglycerinester: Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Anionische Emulgatoren: Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

Amphothere und kationische Emulgatoren: Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonium-glycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodiropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

Fette und Wachse: Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder-stearat kommen hierfür in Frage. Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Perlglanzwachse: Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Konsistenzgeber und Verdickungsmittel: Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Überfettungsmittel: Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Stabilisatoren: Als Stabilisatoren können Metallsalze von Fettsäuren, wie z.B. Magnesium-, Aluminium- und/oder Zinkstearat bzw. -ricinoleat eingesetzt werden.

Polymere: Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und

Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in Cosm.Toil. 108, 95 (1993) aufgeführt.

Siliconverbindungen: Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976).

UV-Lichtschutzfilter und Antioxidantien: Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein.

Als öllösliche Substanzen sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben;
➢ 3-(4'-Trimethylammonium) benzyliden- bornan-2-on-methylsulfat (Mexoryl SO)
➢ 3,3'-(1,4-Phenylendimethin)-bis (7,7- dimethyl-2-oxobicyclo-[2.2.1] heptan-1-methansulfonsäure) and salts (Mexoryl SX)
➢ 3-(4'-Sulfo)-benzyliden-bornan-2-on and salts (Mexoryl SL)
➢ Polymer von N-{(2und 4)- [2-oxoborn-3-yliden)methyl}benzyl]acrylamid (Mexoryl SW)
➢ 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl XL)
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester; ➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4- diyl)diimino] bis(benzoesäure-2- ethylhexylester) (Uvasorb® HEB);
➢ 2,2(-Methylen-bis(6-(2H-benzotriazol-2-yl)-4-(1,1,3,3-tetramethyl-butyl)phenol)(Tinosorb M);
➢ 2,4-Bis[4-(2-ethylhexyloxy)-2-hydroxyphenyl]-6-(4-methoxyphenyl)-1,3,5-triazin(TinosorbS);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX).

Als wasserlösliche UV - Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1 H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Erfindungsgemäß bevorzugt sind UV-Lichtschutzfilter ausgewählt aus dem Anhang VII der europäischen Kosmetik-Gesetzgebung (24th Adapting Comission Directive, 29.Februar 2000)

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pig-mente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996) sowie Parf.Kosm. 3, 11 (1999) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleo-side und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydro-guajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Gegenstand der Erfindung sind somit auch Sonnenschutzmittel dadurch gekennzeichnet, dass sie organische UV-Lichtschutzfilter enthalten, die ausgewählt sind aus der Gruppe, die gebildet wird von 3-Benzylidencampher bzw. 3-Benzylidennorcampher und dessen Derivaten, 4-Amino-benzoesäurederivaten, Zimtsäureestern, Salicylsäureestern, Benzalmalonsäureestern, Benzophe-nonderivaten, Benzoylmethanderivaten, Triazinderivaten, Propan-1,3-dionen, Ketotricyclo(5.2.1.0)-decanderivaten, 2-Phenylbenzimidazol-5-sulfonsäure und deren Salzen sowie Sulfonsäurederivaten des Benzophenons bzw. des 3-Benzylidencamphers.

Die erfindungsgemäßen Sonnenschutzmittel können auch anorganische UV-Lichtschutzpigmente enthalten, die ausgewählt sind aus der Gruppe, die gebildet wird von Titandioxid, Zinkoxid, Eisenoxid, Aluminiumoxid, Ceroxid, Zirkoniumoxid, Silicaten, Bariumsulfat und Zinkstearat.

Die erfindungsgemäßen Sonnenschutzmittel können auch Antioxidantien enthalten, die ausgewählt sind aus der Gruppe, die gebildet wird von Aminosäuren und deren Derivaten, Imidazole (z.B. Urocaninsäure) und deren Derivaten, Peptiden und deren Derivaten, Carotinoiden, Carotinen und deren Derivaten, Chlorogensäuren und deren Derivaten, Liponsäure und deren Derivaten, Aurothioglucose, Propylthiouracil und anderen Thiolen sowie deren Salzen, Dilaurylthiodipropionat, Di-stearylthiodipropionat, Thiodipropionsäure und deren Derivaten, Sulfoximinverbindungen, (Metall)-Chelatoren, alpha-Hydroxysäuren, Huminsäure, Gallensäure, Gallenextrakte, Bilirubin, Biliverdin, EDTA, EGTA und deren Derivaten, ungesättigten Fettsäuren und deren Derivaten, Folsäure und deren Derivaten, Ubichinon, Ubichinol und deren Derivaten, Vitamin C und dessen Derivaten, Tocopherolen und deren Derivaten, Vitamin A und dessen Derivaten, Koniferylbenzoat, Rutinsäure und deren Derivate, alpha-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Superoxid-Dismutase, Zink und dessen Derivaten, Selen und dessen Derivaten sowie Stilbenen und deren Derivaten.

Biogene Wirkstoffe: Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Deodorantien und keimhemmende Mittel: Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Keimhemmende Mittel: Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Enzyminhibitoren: Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

Geruchsabsorber: Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, alpha-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, beta-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien: Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe: adstringierende Wirkstoffe, Ölkomponenten, nichtionische Emulgatoren, Coemulgatoren, Konsistenzgeber, Hilfsstoffe wie z. B. Verdicker oder Komplexierungsmittel und/oder nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z.B. mit Propylenglycol-1,2. Aluminiumhydroxyallantoinat, Aluminiumchloridtartrat, Aluminium-Zirkonium-Trichlorohydrat, Aluminium-Zirko-niumtetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein: entzündungshemmende, hautschützende oder wohlriechende ätherische Öle, synthetische hautschützende Wirkstoffe und/oder öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Filmbildner: Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Antischuppenwirkstoffe: Als Antischuppenwirkstoffe kommen Pirocton Olamin (1-Hydroxy-4-methyl-6-(2,4,4-trimythylpentyl)-2-(1 H)-pyridinonmonoethanolaminsalz), Baypival® (Climbazole), Ketoconazol®, (4-Acetyl-1-{-4-[2-(2.4-dichlorphenyl) r-2-(1 H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

Quellmittel: Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

Insekten-Repellentien: Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Selbstbräuner und Depigmentierungsmittel: Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Hydrotrope: Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

Konservierungsmittel: Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Parfümöle und Aromen: Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, 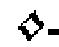 -Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, alpha-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, beta-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Farbstoffe: Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Bakterizide bzw. bakteriostatische Wirkstoffe: Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Schweißabsorbierende Substanzen: Als schweißabsorbierende Substanzen sind zum Beispiel modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierende Substanzen in Mengen von 0,1 bis 30, vorzugsweise 1 bis 20 und insbesondere 2 bis 8 Gew.-% - bezogen auf das Gesamtgewicht der kosmetischen und/oder pharmazeutischen Zubereitung - enthalten.

Die Pigmente liegen in feinteiliger Form in den Pigmentzubereitungen vor und haben dementsprechend üblicherweise mittlere Teilchengrößen von 0,02 bis 5 µm.

Bei den organischen Pigmenten handelt es sich üblicherweise um organische Bunt- und Schwarzpigmente. Anorganische Pigmente können ebenfalls Farbpigmente (Bunt-, Schwarz- und Weißpigmente) sowie Glanzpigmente und die üblicherweise als Füllstoffe eingesetzten anorganischen Pigmente sein.

Als Pigmente, insbesondere mit farbgebenden Eigenschaften können Eisenoxide, Titanoxid, synthetisches Fluorphlogopit und Titanoxid, Glimmer und/oder Zinkoxid eingesetzt werden.

Gegenstand der Erfindung sind auch Lotionen. In einer Alternative enthält oder besteht eine erfindungsgemäße Lotion, insbesondere Sonnenschutzmittel (aus) folgende(n) Komponenten:
erfindungsgemässe Zusammensetzung 1-10%, bevorzugt 4-6%;
Emulgatoren 0.5-5%, bevorzugt 1.5-3%;
Emollients, bevorzugt werden unterschiedliche Emollients eingesetzt deren Anteil in der Summe 20-50%, bevorzugt 25-35% ist;
UV-Filter, bevorzugt werden unterschiedliche Filter eingesetzt deren Anteil in der Summe 10-35%, bevorzugt 20-25% ist;
demineralisiertes Wasser 5-50%, bevorzugt 20-30%;
   sowie weitere Zusatzstoffe, bevorzugt unterschiedliche, ausgewählt aus der Gruppe der oben offenbarten, so dass die Summe aller Inhaltsstoffe 100% ergibt.

In einer weiteren Alternative enthält oder besteht eine erfindungsgemäße Lotion (aus) folgende(n) Komponenten:
erfindungsgemässe Zusammensetzung 0.1-5%, bevorzugt 1-2%;
Emulgatoren 0.01-5%, bevorzugt 0.1-1%;
Emollients, bevorzugt werden unterschiedliche Emollients eingesetzt deren Anteil in der Summe 1-20%, bevorzugt 5-12% ist;
Konsistenzgeber und Verdickungsmittel, bevorzugt werden unterschiedliche Mittel eingesetzt deren Anteil in der Summe 0.5-10%, bevorzugt 2-5% ist;
Rheologiestoffe 0.01-5%, bevorzugt 0.1-1%;
Antioxidationsmittel 0.01-10%, bevorzugt 0.1-1%;
Humectant 1-10%, bevorzugt 2-8%, besonders bevorzugt 4-6%;
demineralisiertes Wasser 50-90%, bevorzugt 75-85%%;
   sowie weitere Zusatzstoffe, bevorzugt unterschiedliche, ausgewählt aus der Gruppe der oben offenbarten, so dass die Summe aller Inhaltsstoffe 100% ergibt.

Gegenstand der Erfindung sind auch Cremes. In einer weiteren Alternative enthält oder besteht eine erfindungsgemäße Creme (aus) folgende(n) Komponenten:
erfindungsgemässe Zusammensetzung 0.1-10%, bevorzugt 2-5%;
Emulgatoren 0.01-5%, bevorzugt 0.1-1%;
Emollients, bevorzugt werden unterschiedliche Emollients eingesetzt, deren Anteil in der Summe 1-30%, bevorzugt 10-20% ist;
Konsistenzgeber und Verdickungsmittel, bevorzugt werden unterschiedliche Mittel eingesetzt deren Anteil in der Summe 0.1-10%, bevorzugt 1.5-5% ist;
Rheologiestoffe 0.01-5%, bevorzugt 0.1-1%;
Humectant 1-10%, bevorzugt 2.5-5%;
demineralisiertes Wasser 50-90%, bevorzugt 70-80%;
   sowie weitere Zusatzstoffe, bevorzugt unterschiedliche, ausgewählt aus der Gruppe der oben offenbarten, so dass die Summe aller Inhaltsstoffe 100% ergibt.

Gegenstand der Erfindung ist auch eine Körper-Butter. In einer weiteren Alternative enthält oder besteht eine erfindungsgemäße Körper-Butter (aus) folgende(n) Komponenten sowie ggf. weitere Hilfsstoffe:
erfindungsgemässe Zusammensetzung 0.1-10%, bevorzugt 1-5%;
Emulgatoren 0.01-5%, bevorzugt 0.1-1%;
Emollients, bevorzugt werden unterschiedliche Emollients eingesetzt, deren Anteil in der Summe 5-40%, bevorzugt 20-25% ist;
Konsistenzgeber und Verdickungsmittel, bevorzugt werden unterschiedliche Mittel eingesetzt deren Anteil in der Summe 0.5-20%, bevorzugt 5-10% ist;
Rheologiestoffe 0.01-5%, bevorzugt 0.1-1%;
Humectant 1-10%, bevorzugt 2.5-7.5%;
demineralisiertes Wasser 40-80%, bevorzugt 60-70%;
Antioxidationsmittel 0.01-5%, bevorzugt 0.1-1%;
   sowie weitere Zusatzstoffe, bevorzugt unterschiedliche, ausgewählt aus der Gruppe der oben offenbarten, so dass die Summe aller Inhaltsstoffe 100% ergibt.

Gegenstand der Erfindung sind auch Produkte der dekorativen Kosmetik, wie zum Beispiel Lippenstift, Lip Gloss, Lidschatten, Puder, Rouge, Make-up, Concealer, Eyeliner, Mascara, Kajal, sowie BB(Blemish Balm)-Erzeugnisse, wie zum Beispiel BB Cremes, BB Foundations, BB-Concealer.

In einer Alternative enthält oder besteht ein erfindungsgemäßes BB (Blemish Balm)-Erzeugnis (aus) folgende(n) Komponenten, so dass die Summe aller Inhaltsstoffe 100% ergibt: Gereinigtes Wasser:bis 100 %, Butylenglykol: 1-10%, bevorzugt 2-6%, Eisenoxide: 0.01-5%, bevorzugt 0.1-1%, Titanoxid: 1-10%, bevorzugt 3-7%, Xanthangummi: 0.01-2%, bevorzugt 0.05-1.0%, Erfindungsgemässe Zusammensetzung: 1-10%, bevorzugt 2-8%,Dinatriumcetearylsulfosuccinat: 0.1-5%, bevorzugt 1-3%, Diethylaminohydroxybenzoylhexylbenzoat: 1-10%, bevorzugt 2-6%, Methylen- bisbenzotriazolyltetramethylbutylphenol: 1-10%, bevorzugt 2-6%,Octinoxat: 1-10%, bevorzugt 2-8%, Bis-ethylhexyloxyphenolmethoxyphenyltriazin: 0.1-5%, bevorzugt 0.5-3%, Dicaprylylcarbonat: 1-20%, bevorzugt 2-15%, Cyclomethicon: 1-10%, bevorzugt 2-8%, Propylencarbonat: 0.1-5%, bevorzugt 0.2-2%, Stearalkoniumhectorit: 0.1-10%, bevorzugt 0.5-3%, Synthetisches Fluorphlogopit und Titanoxid: 0.5-6%, bevorzugt 1-3%, Glimmer: 1-10%, bevorzugt 2-6%, Zinkoxid: 10-30%, bevorzugt 15-25%, Talk: 1-10%, bevorzugt 2-8%, Natriumpolyacrylat: 0.1-5%, bevorzugt 0.5-.26%, %Acrylat/Methacrylamid-Copolymer: 1-10%, bevorzugt 2-6%.

Gegenstand der Erfindung ist auch eine BB (Blemish Balm)-Körpercreme, insbesondere Sonnenschutzmittel. In einer weiteren Alternative enthält oder besteht eine erfindungsgemäße Körpercreme (aus) folgende(n) Komponenten sowie ggf. weitere Hilfsstoffe, so dass die Summe aller Inhaltsstoffe 100% ergibt:
erfindungsgemässe Zusammensetzung 0.1-20%, bevorzugt 1-10%;
Emulgatoren 0.01-5%, bevorzugt 0.1-2%;
Emollients, bevorzugt werden unterschiedliche Emollients eingesetzt, deren Anteil in der Summe 5-40%, bevorzugt 10-30% ist;
Füller 0.5-20%, bevorzugt 1-10% ist;
UV-Filter, bevorzugt werden unterschiedliche Emollients eingesetzt, deren Anteil in der Summe 1-40%, bevorzugt 10-30% ist;
Rheologiestoffe 0.01-5%, bevorzugt 0.1-2%;
Humectant 0.1-10%, bevorzugt 1-5%;
demineralisiertes Wasser 30-80%, bevorzugt 40-50%;
Effektpigmete, bevorzugt werden unterschiedliche Emollients eingesetzt, deren Anteil in der Summe 0.1-10%, bevorzugt 1-7% ist;
Preservative 0.01-5%, bevorzugt 0.1-2%;

Gegenstand der Erfindung ist auch eine BB (Blemish Balm)-Creme, insbesondere Sonnenschutzmittel. In einer weiteren Alternative enthält oder besteht eine erfindungsgemäße Creme (aus) folgende(n) Komponenten sowie ggf. weitere Hilfsstoffe, so dass die Summe aller Inhaltsstoffe 100% ergibt:
erfindungsgemässe Zusammensetzung 0.1-20%, bevorzugt 1-10%;
Emulgatoren 0.01-8%, bevorzugt 0.1-3%;
Emollients, bevorzugt werden unterschiedliche Emollients eingesetzt, deren Anteil in der Summe 5-40%, bevorzugt 10-30% ist;
Füller 0.5-10%, bevorzugt 1-5% ist;
UV-Filter, bevorzugt werden unterschiedliche Emollients eingesetzt, deren Anteil in der Summe 1-40%, bevorzugt 10-20% ist;
Rheologiestoffe 0.01-10%, bevorzugt 0.1-5%;
Humectant 0.1-15%, bevorzugt 2-10%;
demineralisiertes Wasser 20-70%, bevorzugt 30-50%;
Effektpigmete, bevorzugt werden unterschiedliche Emollients eingesetzt, deren Anteil in der Summe 0.1-10%, bevorzugt 1-5% ist;
Farbstoffe, bevorzugt werden unterschiedliche Emollients eingesetzt, deren Anteil in der Summe 0.1-15%, bevorzugt 2-10% ist;
Preservative 0.01-5%, bevorzugt 0.1-2%;

Gegenstand der Erfindung ist auch eine Gesichtsmaske. In einer weiteren Alternative enthält oder besteht eine erfindungsgemäße Gesichtsmaske (aus) folgende(n) Komponenten sowie ggf. weitere Hilfsstoffe:
erfindungsgemässe Zusammensetzung 0.1-10%, bevorzugt 1-5%;
Emulgatoren 0.01-5%, bevorzugt 0.1-1%;
Emollients, bevorzugt werden unterschiedliche Emollients eingesetzt, deren Anteil in der Summe 5-30%, bevorzugt 10-20% ist;
Konsistenzgeber und Verdickungsmittel, bevorzugt werden unterschiedliche Mittel eingesetzt deren Anteil in der Summe 0.5-15%, bevorzugt 2-5% ist;
Rheologiestoffe 0.01-5%, bevorzugt 0.1-1%;
Humectant 1-10%, bevorzugt 2-8%;
demineralisiertes Wasser 50-90%, bevorzugt 65-75%;
   sowie weitere Zusatzstoffe, bevorzugt unterschiedliche, ausgewählt aus der Gruppe der oben offenbarten, so dass die Summe aller Inhaltsstoffe 100% ergibt.

Gegenstand der Erfindung ist auch eine Handcreme. In einer weiteren Alternative enthält oder besteht eine erfindungsgemäße Handcreme (aus) folgende(n) Komponenten sowie ggf. weitere Hilfsstoffe, enthaltend die erfindungsgemäße Zusammensetzung sowie weitere Hilfsstoffe:
erfindungsgemässe Zusammensetzung 0.1-10%, bevorzugt 1-5%;
Emulgatoren 0.01-5%, bevorzugt 0.1-1%;
Emollients, bevorzugt werden unterschiedliche Emollients eingesetzt, deren Anteil in der Summe 1-20%, bevorzugt 2-15%, ist;
Konsistenzgeber und Verdickungsmittel, bevorzugt werden unterschiedliche Mittel eingesetzt deren Anteil in der Summe 0.5-15%, bevorzugt 2-5% ist;
Rheologiestoffe 0.01-5%, bevorzugt 0.1-1%;
Humectant 1-30%, bevorzugt 2 5-20%;
demineralisiertes Wasser 50-90%, bevorzugt 65-75%;
   sowie weitere Zusatzstoffe, bevorzugt unterschiedliche, ausgewählt aus der Gruppe der oben offenbarten, so dass die Summe aller Inhaltsstoffe 100% ergibt.

Gegenstand der Erfindung ist auch ein Serum. In einer weiteren Alternative enthält oder besteht ein erfindungsgemäßes Serum (aus) folgende(n) Komponenten sowie ggf. weitere Hilfsstoffe, enthaltend die erfindungsgemäße Zusammensetzung sowie weitere Hilfsstoffe: erfindungsgemässe Zusammensetzung 0.1-10%, bevorzugt 1-5%;
Emulgatoren 0.01-5%, bevorzugt 0.1-1%;
Emollients, bevorzugt werden unterschiedliche Emollients eingesetzt, deren Anteil in der Summe 1-20%, bevorzugt 2-10%;
Konsistenzgeber und Verdickungsmittel, bevorzugt werden unterschiedliche Mittel eingesetzt deren Anteil in der Summe 0.1-10%, bevorzugt 0.5-2%;
Rheologiestoffe 0.01-5%, bevorzugt 0.1-1%,;
Humectant 0.1-10%, bevorzugt 1-5%;
demineralisiertes Wasser 60-95%, bevorzugt 80-90%;
   sowie weitere Zusatzstoffe, bevorzugt unterschiedliche, ausgewählt aus der Gruppe der oben offenbarten, so dass die Summe aller Inhaltsstoffe 100% ergibt.

In einer weiteren Alternative werden erfindungsgemäße kosmetische Erzeugnisse, insbesondere der dekorativen Kosmetik, ausgewählt aus folgenden Tabellen:
Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen. Man kann die Mittel nach der Phaseninversionstemperatur-Methode herstellen.

Die erfindungsgemäße Zusammensetzung, der erfindungsgemäße Formkörper sowie das erfindungsgemäße Verfahren zeigen hervorragende sensorische Eigenschaften und ermöglichen die Verwendung nachhaltiger und/oder nachwachsender Rohstoffe. Dabei handelt es sich um Rohstoffe die nicht petrochemisch hergestellt werden und die pflanzlichen oder tierischen Ursprungs sind und außerhalb des Ernährungsbereiches (Nahrungs- und Futtermittel) stofflich oder energetisch genutzt werden können.

Gegenstand der vorliegenden Erfindung sind somit auch kosmetische Erzeugnisse die die Anforderungen gemäß Ecocert für Naturkosmetik und Bio-Naturkosmetik erfüllen:
Ecocert Naturkosmetik:
   Mindestens 95% aller Inhaltsstoffe sind natürlichen Ursprungs.
   Mindestens 5% aller Inhaltsstoffe im Endprodukt stammen aus biologischem Anbau
   Mindestens 50% der eingesetzten pflanzlichen Stoffe stammen aus biologischem Anbau Synthetische Farb- und Duftstoffe, synthetische Fette, Öle, Silikone und Erdölprodukte sind nicht erlaubt
   Keine Tierversuche mit dem fertigen Produkt
   Einsatz tierischer Rohstoffe nur von lebenden Tieren
   Zur Konservierung sind nur bestimmte Stoffe erlaubt
   Verzicht auf gentechnisch manipulierte Rohstoffe
   Keine radioaktive Bestrahlung zur Entkeimung von Rohstoffen oder Produkten
Ecocert Biologische Naturkosmetik: Im Vergleich zur bloßen Naturkosmetik sind hier höhere Anteile von Zutaten aus kontrolliert biologischem Anbau vorgeschrieben:
   Mindestens 95% aller Inhaltsstoffe sind natürlichen Ursprungs
   Mindestens 10% aller Inhaltsstoffe stammen aus biologischem Anbau
   Mindestens 95% der eingesetzten pflanzlichen Stoffe stammen aus biologischem Anbau Synthetische Farb- und Duftstoffe, synthetische Fette, Öle, Silikone und Erdölprodukte sind nicht erlaubt
   Keine Tierversuche mit dem fertigen Produkt
   Einsatz tierischer Rohstoffe nur von lebenden Tieren
   Zur Konservierung sind nur bestimmte Stoffe erlaubt
   Verzicht auf gentechnisch manipulierte Rohstoffe Keine radioaktive Bestrahlung zur Entkeimung von Rohstoffen oder Produkten

### Beispiele:

### 1. Erfindungsgemäß

Sucroseester wurde gemäß Beispiel 1 der EP 1 811 91 B1 hergestellt. Die Aufarbeitung erfolgte jedoch ohne Deaktivierung des Katalysators. Es wurde bei 100°C jeweils als Emollient Cetyl Palmitat, Hexyldecyl Stearat und gehärtetes Palmö zugegeben I.

Nach Dekantieren bei 100°C **mittels** Zentrifuge oder Dekanter erfolgte eine Bleiche mittels 35%iger H2O2-Lösung. Bei Raumtemperatur lag ein fast farbloser Feststoff vor.

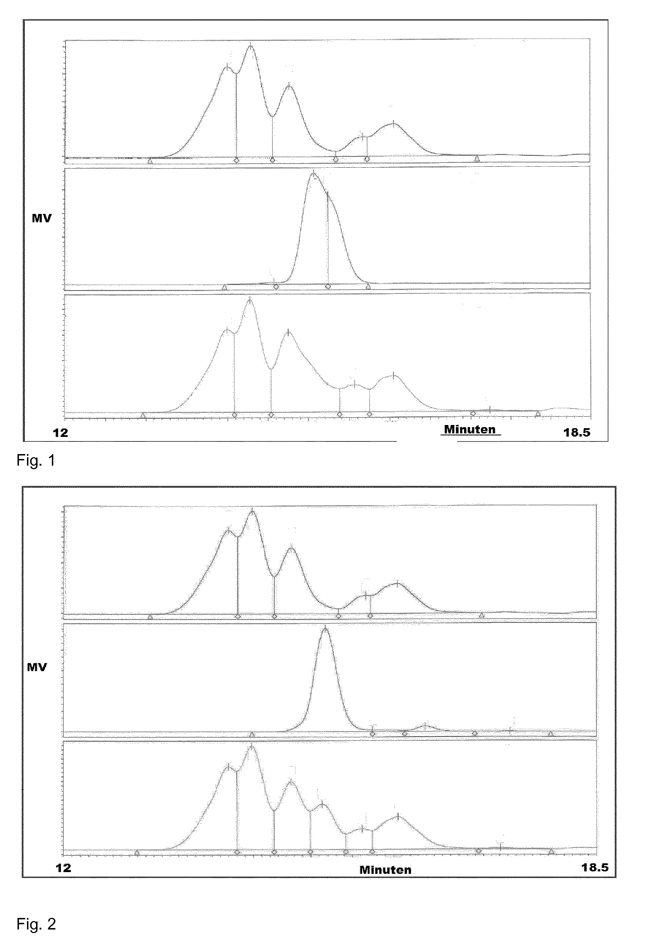

GPC-Analysen zeigten keine Änderung der Kohlehydratpartialesterverteilung zwischen Rohund Endprodukt:
Emollient :
   Cetyl Palmitat in Fig. 1, Hexyldecyl Stearat in Fig. 2 und gehärtetes Palmöl in Fig. 3.

Die Fig. zeigen jeweils in der oberen Abbildung das GPC des jeweiligen Rohprodukts aus Schritt i) des erfindungsgemässen Verfahrens, die mittlere Abbildung zeigt das GPC des jeweiligen reinen Emollients und die untere Abbildung das GPC des jeweiligen Endprodukts.

Die aus dem Endprodukt hergestellten Pastillen waren hart aber nicht spröde. Es wurde kein "Schwitzen" beobachtet, auch bei Lagerung länger als eine Woche bei 40°C. Die Pastillen verklebten auch nicht im Lagertest.

### 2. Vergleichsbeispiel

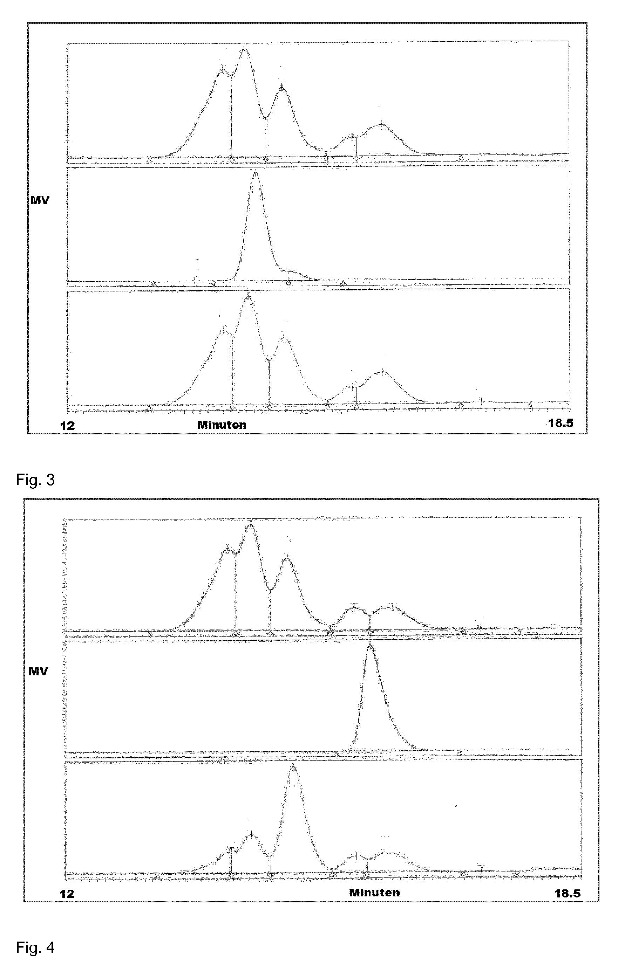

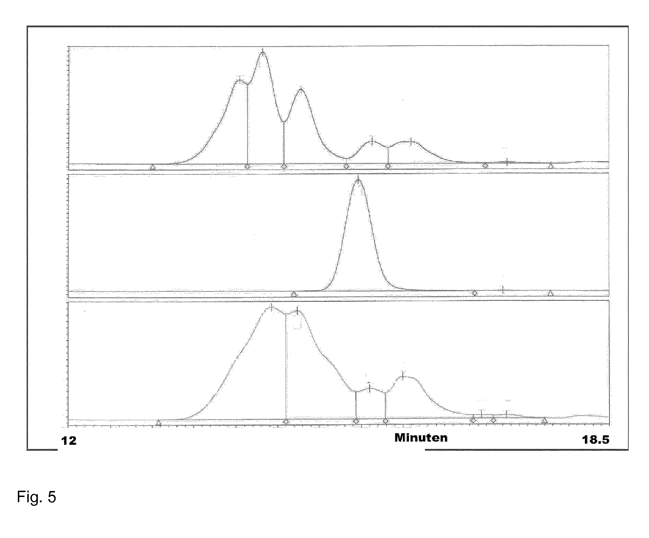

Die Vergleichsbeispiele wurden analog Beispiel 1 durchgeführt. Als Emollients wurden aber jeweils Behenyl Alkohol in Fig 4. sowie Capryl- / Caprin Triglyceride (Myritol 312) in Fig. 5 eingesetzt.

GPC-Analysen zeigten unterschiedliche Kohlehydratpartialesterverteilungen jeweils zwischen Roh- und Endprodukt.

## Patentansprüche

1. Zusammensetzung enthaltend:
a1) Kohlehydratpartialester als Produkt einer Veresterung mindestens eines Kohlehydrats mit Acylkomponenten der Formel R1-COO,
b1) Alkylester der Formel R1-COO-R2 und
c1) Esteröle der Formel R1-COO-R3.
sowie ggf. Kohlehydrate, Katalysator, Fettsäuren und/oder Fettseifen;
wobei die Esteröle c1) eine Alkoholkomponente R3 ausgewählt aus der Gruppe enthaltend
i) verzweigte und unverzweigte Alkohole mit C8 - C22 oder
ii) Polyalkohole oder
iii) Mischungen davon
besitzen, welche vollständig verestert sind mit einer Acylkomponente R1 ausgewählt aus der Gruppe von Mono- und Disäuren der Kettenlänge C6 - C22 oder beliebige Kombinationen.

2. Zusammensetzung enthaltend:
a2) Kohlehydratpartialester mit einem durchschnittlichen Veresterungsgrad von 1 - 4,
b2) organische Alkylester mit Acylkomponente mit 6 - 22 Kohlenstoffatomen,
c2) Esteröle mit einer linearen Acylkomponente mit 6-22 Kohlenstoffatomen und einer linearen Alkoholkomponente mit 8-22 Kohlenstoffatomen,
sowie ggf. Kohlehydrate, Katalysator, Fettsäuren und/oder Fettseifen.

3. Zusammensetzung nach einem der vorangehenden Ansprüche **dadurch gekennzeichnet, dass** das Produkt a1) oder a2) ein Kohlehydratpartialester einer Glycose mit Acylkomponente von b1) oder b2) ist.

4. Zusammensetzung nach Anspruch 3 **dadurch gekennzeichnet, dass** die Glykosen Mono- und/oder Disaccharide sind.

5. Zusammensetzung nach einem der vorangehenden Ansprüche **dadurch gekennzeichnet, dass** die Alkoholkomponente des Esters b1) oder b2) eine C1 bis C3 Alkoholeinheit ist.

6. Zusammensetzung nach einem der vorangehenden Ansprüche **dadurch gekennzeichnet, dass** mindestens eine Acylkomponente des Esters a1), b1), c1), a2), b2) oder c2) ein Fettsäurerest mit 16 und/oder 18 Kohlenstoffatomen ist.

7. Zusammensetzung nach einem der vorangehenden Ansprüche **dadurch gekennzeichnet, dass** die Alkoholkomponente der Esteröle c1) oder c2) ein Fettalkohol mit 16 und/oder 18 Kohlenstoffatomen ist.

8. Zusammensetzung nach einem der Ansprüche 1 bis 6 **dadurch gekennzeichnet, dass** die Alkoholkomponente der Esteröle c1) ein Guerbetalkohol mit 16 bis 20 Kohlenstoffatomen oder ein Polyalkohol ausgewählt aus der Gruppe enthaltend: Ethylenglykol, Propylenglykol, Butylenglykol, Neopentylglykolen , Glycerin ist, welche vollständig verestert sind ist.

9. Zusammensetzung nach einem der vorangehenden Ansprüche **dadurch gekennzeichnet, dass** Kohlehydradpartialester
Monoester in einem Anteil von 0% bis 20%,
Diester in einem Anteil von 10% bis 40%,
Triester in einem Anteil von 20% bis 50% und
Tetraester in einem Anteil von 15% bis 40% enthält,
wobei die Summe 100% ist.

10. Zusammensetzung nach einem der vorangehenden Ansprüche **dadurch gekennzeichnet, dass** das Verhältnis von Monoester : Diester : Triester : Tetraester zwischen 0.5 und 1.5 : zwischen 3.0 und 4.0 : zwischen 4.0 und 5.0 : zwischen 3.0 und 4.0 ist.

11. Formkörper enthaltend oder bestehend aus einer der Zusammensetzung nach Anspruch 1 - 10.

12. Verfahren zur Herstellung einer Zusammensetzung nach einem der Ansprüche 1 - 10 oder eines Formkörpers nach Anspruch 11 **gekennzeichnet durch** folgende Schritte:
i) Ver- bzw. Umesterung eines Kohlehydrats mit einem Alkylester b1) oder b2) in Gegenwart eines Katalysators,
ii) Zugabe von Esterölen c1 oder c2),
iii) Abtrennung des Restzuckers vomRohprodukt, ggf.
iv) Bleichen der abgetrennten flüssigen Phase enthaltend das Rohprodukt und ggf.
v) Herstellen eines Formkörpers.

13. Verfahren nach Anspruch 12 **dadurch gekennzeichnet, dass** keine Deaktivierung des Katalysators erfolgt.

14. Verfahren nach Anspruch 12 **dadurch gekennzeichnet, dass** das Produkt aus Schritt i) dieselbe Kohlehydratpartialesterverteilung aufweist wie eines der Produkte aus Schritt iii), iv) oder v).

15. Kosmetisches und/oder pharmazeutisches Erzeugnis und/oder Lebensmittel enthaltend eine Zusammensetzung nach einem der Ansprüche 1 - 10 oder einen Formkörper nach Anspruch 11 oder ein Produkt, hergestellt in einem Verfahren gemäß einem der Ansprüche 12 - 14.
